(19) Europäisches Patentamt
European Patent Office
Office européen des brevets

(11) Numéro de publication: **0 194 178**
**A1**

(12) # DEMANDE DE BREVET EUROPEEN

(21) Numéro de dépôt: **86400301.7**

(22) Date de dépôt: **12.02.86**

(51) Int. Cl.⁴: **A 61 K 31/44**
// (A61K31/44, 31:135)

(30) Priorité: **26.02.85 FR 8502727**

(43) Date de publication de la demande: **10.09.86**
**Bulletin 86/37**

(84) Etats contractants désignés: **BE CH DE FR GB IT LI LU NL SE**

(71) Demandeur: **SYNTHELABO, 58, rue de la Glacière, F-75621 Paris Cedex 13 (FR)**

(72) Inventeur: **Cavero, Icilio, 8, Rue Gabriel Fauré, F-94000 Créteil (FR)**

(74) Mandataire: **Thouret-Lemaitre, Elisabeth et al, Service Brevets - SYNTHELABO 58, rue de la Glacière, F-75621 Paris Cedex 13 (FR)**

(54) **Compositions pharmaceutiques à base de bétaxolol et de nifédipine.**

(57) Composition pharmaceutique contenant une association de betaxolol et de nifédipine.

EP 0 194 178 A1

La présente invention a pour objet des compositions pharmaceutiques à base de betaxolol et de nifédipine destinées au traitement de maladies cardiovasculaires, plus particulièrement de l'hypertension.

Le bétaxolol de formule

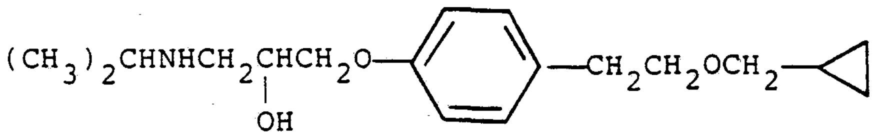

est connu pour ses propriétés β-bloquantes.

La nifédipine de formule

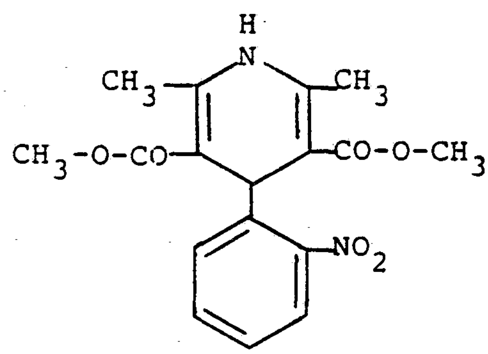

est connue pour ses propriétés antihypertensives et d'inhibiteur calcique.

La Demanderesse a constaté que, de manière surprenante, il existe un effet de synergie important entre les propriétés antihypertensives des deux composés lorsqu'on les associe.

Les compositions pharmaceutiques de l'invention ont été soumises à une série d'essais pharmacologiques qui révèlent leurs intéressantes propriétés dans le domaine cardiovasculaire.

Le test utilisé est le suivant : des rats spontanément hypertendus, mâles et vigiles, sont utilisés. Leurs pression artérielle et leur fréquence cardiaque sont enregistrées en continu à partir de l'artère caudale préalablement cathétérisée pendant une courte période d'anesthésie légère à l'éther. Après une période de stabilisation (2 h environ) des paramètres cardiaques, les animaux reçoivent par voie orale, sous un volume de 5 ml/kg, le solvant (eau distillée), le bétaxolol (2,5 mg/kg), la nifédipine (10 mg/kg) ou l'association bétaxolol/nifédipine (2,5/10 mg/kg).

L'administration orale de bétaxolol (2,5 mg/kg) n'a pas d'effet significatif sur la pression artérielle moyenne et diminue légèrement la fréquence cardiaque des animaux.
La nifédipine (10 mg/kg, p.o.) diminue d'environ 20 % la pression artérielle moyenne pendant les 2 premières heures qui suivent l'administration, cette action n'étant plus que de 12 %, 4 heures après le traitement. Cet effet hypotenseur est accompagné d'une importante tachycardie.
L'association bétaxolol/nifédipine (2,5/10 mg/kg p.o.) engendre une chute de la pression artérielle moyenne plus importante en amplitude et en durée que celle produite par la nifédipine seule. En pourcentage, la diminution est encore de 29 % après 4 heures, alors que la diminution obtenue avec la nifédipine seule n'est plus que de 12 % après ce même temps.
De plus, alors que la fréquence cardiaque est significativement augmentée par la nifédipine seule, à la dose utilisée (10 mg/kg), aucune tachycardie n'apparaît lors de son association avec le bétaxolol (2,5 mg/kg).

Les résultats sont donnés dans le tableau suivant.

Tableau

| Temps après traitement (min) | PLACEBO (n=4) | ΔPAM (mmHg) BET 2,5 (n=3) | NIF 10 (n=6) | BET 2,5 + NIF 10 (n=11) |
|---|---|---|---|---|
| 15 | -3 ± 2 | -7 ± 7 | -38 ± 2 | -48 ± 2 |
| 30 | 2 ± 4 | -5 ± 3 | -34 ± 3 | -51 ± 3 |
| 45 | 4 ± 3 | -8 ± 4 | -35 ± 4 | -50 ± 4 |
| 60 | 4 ± 3 | -8 ± 3 | -34 ± 5 | -52 ± 4 |
| 90 | 3 ± 3 | -2 ± 2 | -37 ± 4 | -54 ± 4* |
| 120 | -2 ± 2 | 0 ± 0 | -36 ± 6 | -54 ± 5* |
| 150 | 6 ± 2 | 5 ± 3 | -32 ± 3 | -56 ± 5* |
| 180 | -2 ± 4 | 7 ± 3 | -29 ± 3 | -53 ± 6* |
| 210 | -6 ± 5 | 3 ± 3 | -28 ± 6 | -52 ± 5* |
| 240 | -4 ± 5 | 5 ± 5 | -23 ± 5 | -55 ± 5* |

*Effet antihypertenseur significativement ($p<0,05$ ; t-test) plus important que celui correspondant à la somme des effets de chacun des composés administré seul.

BET = bétaxolol

NIF = nifédipine

ΔPAM = diminution de la pression artérielle moyenne par rapport à celle des témoins (valeur initiale: 187 ± 5 mmHg)

n = nombre d'animaux.

Ces résultats montrent qu'il existe une synergie entre les effets antihypertenseurs de la nifédipine et du bétaxolol.

Les compositions pharmaceutiques de l'invention peuvent contenir de 1 à 10 mg de bétaxolol et de 3 à 30 mg de nifédipine, par unité de prise.

Elles peuvent être présentées sous toute forme appropriée pour l'administration par voie orale ou parentérale, en association avec tout excipient pharmaceutique approprié.

Les compositions pharmaceutiques de l'invention peuvent être utilisées pour le traitement de diverses affections cardiovasculaires, en particulier l'hypertension, l'angine de poitrine et l'athérosclérose.

La posologie quotidienne est telle que l'on administre de 1 à 20 mg de bétaxolol et de 3 à 60 mg de nifédipine.

Revendication

1. Composition pharmaceutique contenant une association de bétaxolol et de nifédipine.

2. Composition selon la revendication 1, caractérisée en ce qu'elle contient de 1 à 10 mg de bétaxolol et de 3 à 30 mg de nifédipine.

Office européen des brevets

# RAPPORT DE RECHERCHE EUROPEENNE

0194178

Numero de la demande

EP 86 40 0301

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
| --- | --- | --- | --- |
| P,X | EP-A-0 165 450 (POSANSKI)<br>* Page 12, lignes 1-5; revendication 1 *<br>--- | 1,2 | A 61 K 31/44 //<br>(A 61 K 31/44<br>A 61 K 31:135) |
| X | CHEMICAL ABSTRACTS, vol. 89, no. 21, 20 novembre 1978, pages 73-74, no. 173865b, Columbus, Ohio, US; K. AOKI et al.: "Antihypertensive effect of cardiovascular calcium ion-antagonist in hypertensive patients in the absence and presence of beta-adrenergic blockade", & AM. HEART J. 1978, 96(2), 218-26<br>* Abrégé *<br>----- | 1,2 | |
| | | | DOMAINES TECHNIQUES RECHERCHES (Int. Cl.4) |
| | | | A 61 K |

Le présent rapport de recherche a été établi pour toutes les revendications

| Lieu de la recherche | Date d'achèvement de la recherche | Examinateur |
| --- | --- | --- |
| LA HAYE | 29-05-1986 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82